# EUROPEAN PATENT APPLICATION

(11) **EP 3 040 072 A1**
(43) Date of publication of application: **06.07.2016**
(21) Application number: 14840047.6
(22) Date of filing: 27.08.2014
(51) Int. Cl.: A61K 31/506, A61P 1/16, A61P 35/00, A61P 43/00

(54) **PHARMACEUTICAL COMPOSITION HAVING PYRIMIDINE COMPOUND AS ACTIVE INGREDIENT**

(30) Priority: 28.08.2013 JP 2013176355
(71) Applicant: Astellas Pharma Inc., Chuo-ku Tokyo 103-8411 (JP)
(72) Inventor: FUTAMI, Takashi, Tokyo 103-8411 (JP); TAKESHITA, Rumi, Tokyo 103-8411 (JP)
(74) Representative: Hoffmann Eitle
(86) International application number: PCT/JP2014/072349
(87) International publication number: WO 2015/030021

(57) **Abstract**

[Problem] To provide a pharmaceutical composition for treating FGFR4-realted cancer, FGF19-related cancer, or FGF19 gene amplification positive liver cancer. [Solution] The inventors perfected the present invention through study of compounds having FGFR4 inhibitory effect, verifying that specific pyrimidine compounds have FGFR4 inhibitory effect, and that pharmaceutical compositions having these compounds as active ingredients have therapeutic effect against FGFR4-related cancer, or in another mode against FGF19-related cancer, or in yet another mode against FGF19 gene amplification positive liver cancer.

## Description

### Technical Field

The present invention relates to a pharmaceutical composition for treating FGFR4-related cancer, which comprises a pyrimidine compound or a pharmaceutically acceptable salt thereof as an active ingredient, in another embodiment, a pharmaceutical composition for treating FGF19-related cancer, which comprises a pyrimidine compound or a pharmaceutically acceptable salt thereof as an active ingredient, and in still another embodiment, a pharmaceutical composition for treating FGF19 gene amplification-positive liver cancer, which comprises a pyrimidine compound or a pharmaceutically acceptable salt thereof as an active ingredient.

### Background Art

The signaling pathway induced by fibroblast growth factors (FGFs) and receptors thereof, fibroblast growth factor receptors (FGFRs), is one of signaling pathways having the most important functions in the course of development from early embryogenesis to the formation of various organs. There are 18 genes encoding FGF and 4 genes encoding FGFR (FGFR1 to FGFR4), which are expressed in various cells and involved in cell growth, differentiation, and survival. It has been known that FGF19 is a physiological ligand for FGFR4, secreted from the small intestine after diets, and controls the catabolism of cholesterol through the FGFR4 in the liver (Nat Rev Drug Discov. 2009 Mar; 8 (3) :235-53). Further, in recent years, it has been found that FGF19 is involved in a plurality of cancers such as liver cancer and colon cancer (Cancer Cell. 2011,19(3): 347-58, Oncogene. 2008 Jan 3; 27(1): 85-97, Adv Exp Med Biol. 2012; 728: 183-94).

Primary liver cancer is classified into hepatocellular carcinoma, cholangiocellular carcinoma, hepatoblastoma, and the like according to a tissue classification, and hepatocellular carcinoma accounts for about 90% of primary liver cancer. The hepatocellular carcinoma is one of intractable cancer having poor prognosis cancer, and in particular, five-year survival rate of the patients with surgically unresectable liver cancer, which accounts for 70% of patients diagnosed with hepatocellular carcinoma, is about 10%. Even after surgical resection, about 25% of the patients relapse within one year and about 80% of the patients relapse within five years (Nat. Rev. Gastroenterol. Hepatol. 6,423-432(2009), Cancer MPact 2010, Treatment Architecture US (2011), National Cancer Institute, Physician Data Query (2012), GLOBOCAN 2008, IARC. http: //globocan.iarc.fr/). In Japan, the United States, and Europe (France, Germany, Italy, Spain, and the United Kingdom), there are about 100,000 patients to be treated for liver cancer (Cancer MPact 2010). Further, the patients to be treated for liver cancer can often be seen especially in Southeast Asia and the number of the newly diagnosed patients worldwide is estimated to be about 750,000 (GLOBOCAN 2008, International Agency for Research on Cancer).

Only sorafenib has been approved as a molecular target drug for surgically unresectable liver cancer, but the survival rate remains in an extension from 7.9 months to 10.7 months (N Engl J Med. 2008 Jul 24; 359(4): 378-90).

Recently, there has been a report suggesting that FGF19 gene amplification is involved in hepatocellular carcinoma (Cancer Cell. 2011, 19(3): 347-58). According to this report, gene amplification has been identified in chromosome 11q13.3 region in 14% of the hepatocellular carcinoma, and FGF19 genes are contained in this region. Further, HuH-7 cell is FGF19 gene amplification-positive hepatocellular carcinoma cell line, and the cell growth thereof has been inhibited by adding FGF19 neutralizing antibodies to a culture medium. Further, administration of FGF19 neutralizing antibodies has inhibited the tumor growth in nude mice bearing subcutaneous HuH-7 tumors (Cancer Cell. 2011, 19(3): 347-58). Furthermore, there have been other reports suggesting that FGF19 relates to liver cancer, such as the reports that FGF19-expressing transgenic mice spontaneously develop liver cancer (Am J Pathol. 2002 Jun; 160(6): 2295-307), that FGF19 is highly expressed in tumor sites of liver cancer patients, that the high expression group of patients has a poor prognosis (BMC Cancer. 2012 Feb 6; 12(1): 56), and the like.

On the other hand, there has been a suggestion that FGF19 is involved in liver carcinogenesis through FGFR4, since liver carcinogenesis in FGF19 overexpressing transgenic mice is inhibited by FGFR4 knockdown (PLoS One. 2012; 7(5): e36713). Further, it has been shown that the cell growth is inhibited by the suppression of FGFR4 expression in FGF19 gene amplification-positive hepatocellular carcinoma cell lines by an FGFR4 RNAi, or inhibiting the function of FGFR4 by adding FGFR4 neutralizing antibody to a culture medium (Cancer Discov. 2012 Dec; 2(12): 1118-33, BMC Cancer. 2012 Feb 6; 12: 56, PLoS One. 2012; 7(5): e36713). These reports suggest that an FGFR4 inhibitor has a potential to be effective in the treatment of hepatocellular carcinoma patients having FGF 19 gene amplification.

Recently, there has been reports on the importance of FGF signaling in the development of various types of cancer, and thus, the development of selective FGFR- or FGF inhibitors are in progress (Nature Reviews Cancer 2010,10,116-129, J. Med. Chem. 2011,54,7066-7083, AACR 2011, No. 1643 AstraZeneca).

It has been reported that a compound of the following formula (X1) exhibits various types of kinase inhibition and is useful as an agent for treating cancer and vascular disorders including myocardial infarction (Patent Document 1). Table 2 in the document discloses the test results of kinase inhibition with respect to Yes, VEGFR, EphB4, PDGFRβ, and FGFR1 by some of the compounds, and also discloses that IC₅₀ values for the FGFR1 inhibitory activity is higher than 1000 nM and the activity is also lower than in the other kinase activity inhibition. In addition, in the document, there is no disclosure of a compound which is an active ingredient of the pharmaceutical composition of the present invention.

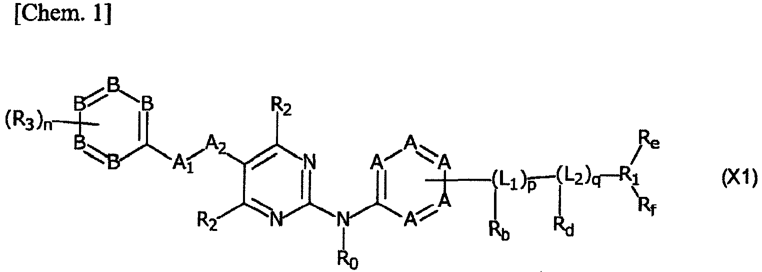

(In this formula, each A is CH, N, or the like; each B is CH or the like; A₁ is O, CR₂, or the like; A₂ is NR, O, or the like; R₀ is H or the like; R is H or the like; L₁ is a bond, O, or the like; L₂ is a bond, C₁-C₆ alkyl, or the like; R₁ is a 3- to 6-membered heterocyclic ring or the like; and Rₑ and R_{f} are H, C₁-C₆ alkyl, hydroxyalkyl, or the like. For the other symbols, refer to the publication.)

It has been reported that a compound of the following formula (X2) exhibits an Ab1 inhibitory action and is useful against various cancers (Patent Document 2). However, in the document, there is no specific description on an FGFR inhibitory action. In addition, there is no disclosure of a compound which is the active ingredient of the pharmaceutical composition of the present invention.

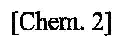

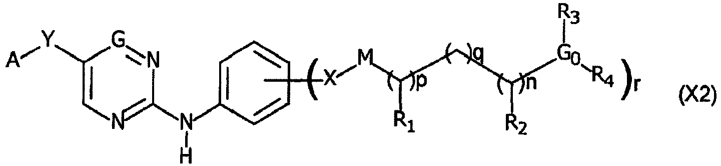

(In this formula, G is CH or the like; A is 3-hydroxyphenyl or the like; and Y is vinyl or ethylene. For the other symbols, refer to the publication.)

It has been reported that a compound of the following formula (X3) has an inhibitory action on various kinases including Src, VEGFR2, Yes, Fyn, Lck, Ab1, PDGFR, EGFR, and RET and can be used for the treatment of cancer, vascular disorders, and the like (Patent Document 3). However, there is no disclosure on an FGFR inhibitory action in the document. In addition, in the document, there is no disclosure of a compound which is the active ingredient of the pharmaceutical composition of the present invention.

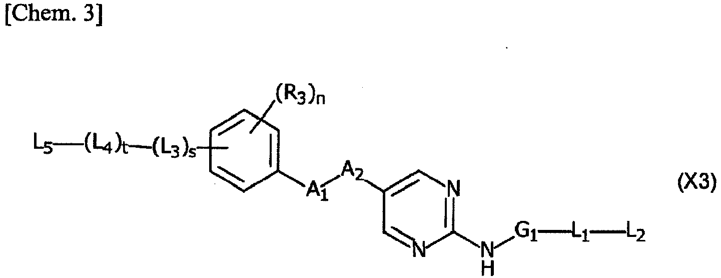

(In this formula, G₁ represents aryl which may be substituted, heteroaryl which may be substituted, or the like; L₁ represents O, SO, SO₂, alkyl which may be substituted, or the like; L₂ represents alkyl which may be substituted, a heterocyclic ring, or the like; A₁ represents a bond, O, C(Rₐ)₂, or the like; A₂ represents NRₐ, O, or the like; and Rₐ represents H or the like. For the other symbols, refer to the publication.)

A document published after the earliest priority date of the present application (Patent Document 4) discloses that a compound of the following formula (X4) is useful as an agent for treating various cancers related to FGFR1, FGFR2, and/or FGFR3, such as lung cancer and hormone therapy-resistant breast cancer which relate to FGFR1, stomach cancer, triple negative breast cancer, and endometrial cancer which are relate to FGFR2, and bladder cancer and glioblastoma which are relate to FGFR3. In addition, in the document, a compound which is the active ingredient of the pharmaceutical composition of the present invention is specifically described as an Example compound, but an action on an inhibitory action on FGFR4 and liver cancer is not disclosed.

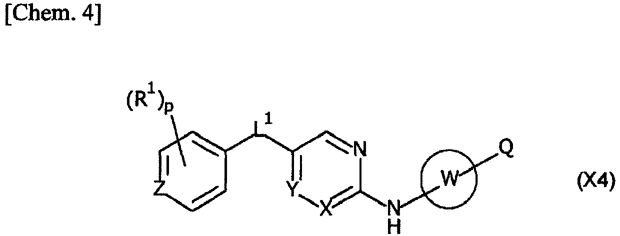

### (The symbols in the formula, refer to the publication.)

### Related Art

### Patent Document

[Patent Document 1] Pamphlet of International Publication WO 2006/101977
[Patent Document 2] Pamphlet of International Publication WO 2007/056075
[Patent Document 3] Pamphlet of International Publication WO 2008/008234
[Patent Document 4] Pamphlet of International Publication WO 2013/129369

### Disclosure of Invention

### Problems to Be Solved by the Invention

A pharmaceutical composition for treating FGFR4-related cancer, in another embodiment, a pharmaceutical composition for treating FGF19-related cancer; and in still another embodiment, a pharmaceutical composition for treating FGF19 gene amplification-positive liver cancer are provided.

### Means for Solving the Problems

The present inventors have conducted extensive investigations on a compound having an inhibitory action on FGFR4 for the purpose of creating a pharmaceutical composition for treating FGFR4-related cancer, in another embodiment, a pharmaceutical composition for treating FGF19-related cancer, and in still another embodiment, a pharmaceutical composition for treating FGF19 gene amplification-positive liver cancer. As a result, they have found that a specific pyrimidine compound or a pharmaceutically acceptable salt thereof has an excellent inhibitory action on FGFR4, and a pharmaceutical composition comprising the compounds as an active ingredient is useful as a pharmaceutical composition for treating FGFR4-related cancer, in another embodiment, a pharmaceutical composition for treating FGF19-related cancer, and in another embodiment, a pharmaceutical composition for treating FGF19 gene amplification-positive liver cancer, thereby completing the present invention.

That is, the present invention relates to a pharmaceutical composition for treating FGFR4-related cancer, in another embodiment, a pharmaceutical composition for treating FGF19-related cancer, and in still another embodiment, a pharmaceutical composition for treating FGF19 gene amplification-positive liver cancer, which comprises 5-[(2,6-difluoro-3,5-dimethoxybenzyl)oxy]-N-{3-methoxy-4-[4-(4-methylpiperazin-1-yl)piperidin-1-yl]phenyl}pyrimidin-2-amine (which is hereinafter referred to as "Compound A" in some cases), 2-[4-({5-[(2,6-difluoro-3,5-dimethoxybenzyl)oxy]pyrimidin-2-yl}amino)-1H-pyrazol-1-yl]ethanol (which is hereinafter referred to as "Compound B" in some cases), (2R)-3-[4-({5-[(2,6-difluoro-3,5-dimethoxybenzyl)oxy]pyrimidin-2-yl}amino)-1H-pyrazol-1-yl]propane-1,2-diol (which is hereinafter referred to as "Compound C" in some cases), 5-[(2,6-difluoro-3,5-dimethoxybenzyl)oxy]-N-[4-(4-methylpiperazin-1-yl)phenyl]pyrimidin-2-amine (which is hereinafter referred to as "Compound D" in some cases), or 5-[(2,6-difluoro-3,5-dimethoxybenzyl)oxy]-N-{1-methyl-5-[(4-methylpiperazin-1-yl)methyl]-1H-pyrazol-3-yl}pyrimidin-2-amine (which is hereinafter referred to as "Compound E" in some cases), or a pharmaceutically acceptable salt thereof, and an excipient.

In addition, Compound A, Compound B, Compound C, Compound D, and Compound E of the pharmaceutical composition of the present invention are the compounds which are described specifically in the above mentioned Patent Document 4.

Furthermore, the present invention relates to an agent for treating FGFR4-related cancer, in another embodiment, an agent for treating FGF19-related cancer, and in another embodiment, an agent for treating FGF19 gene amplification-positive liver cancer, which comprises Compound A, Compound B, Compound C, Compound D, or Compound E, or a pharmaceutically acceptable salt thereof.

Furthermore, the present invention relates to use of Compound A, Compound B, Compound C, Compound D, or Compound E, or a pharmaceutically acceptable salt thereof for the manufacture of a pharmaceutical composition for treating FGFR4-related cancer, in another embodiment, a pharmaceutical composition for treating FGF19-related cancer, and in still another embodiment, a pharmaceutical composition for treating FGF 19 gene amplification-positive liver cancer; use of Compound A, Compound B, Compound C, Compound D, or Compound E, or a pharmaceutically acceptable salt thereof for treating FGFR4-related cancer, in another embodiment, for treating FGF19-related cancer, and in still another embodiment, for treating FGF19 gene amplification-positive liver cancer; Compound A, Compound B, Compound C, Compound D, or Compound E, or a pharmaceutically acceptable salt thereof for treating FGFR4-related cancer, in another embodiment, for treating FGF19-related cancer, and in still another embodiment, for treating FGF19 gene amplification-positive liver cancer; and a method for treating FGFR4-related cancer, in another embodiment, a method for treating FGF19-related cancer, and in still another embodiment, a method for treating FGF19 gene amplification-positive liver cancer, which comprises administering to a subject an effective amount of Compound A, Compound B, Compound C, Compound D, or Compound E, or a pharmaceutically acceptable salt thereof. Further, the "subject" is a human or the other animals in need of treatment thereof, and in a certain embodiment, a human in need of treatment thereof.

### Effects of the Invention

Compound A, Compound B, Compound C, Compound D, or Compound E, or a pharmaceutically acceptable salt thereof, which is the active ingredient of the pharmaceutical composition of the present invention, has an inhibitory action on FGFR4, and can be used as an active ingredient of a pharmaceutical composition for treating FGFR4-related cancer, in another embodiment, a pharmaceutical composition for treating FGF19-related cancer, and in still another embodiment, a pharmaceutical composition for treating FGF19 gene amplification-positive liver cancer.

### Embodiments for Carrying Out the Invention

Hereinbelow, the present invention will be described in detail.

The chemical structure of 5-[(2,6-difluoro-3,5-dimethoxybenzyl)oxy]-N-{3-methoxy-4-[4-(4-methylpiperazin-1-yl)piperidin-1-yl]phenyl}pyrimidin-2-amine (Compound A) is shown below.

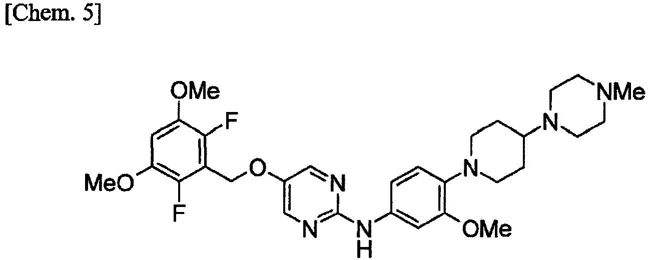

(In the formula, Me represents methyl. In the formulae set forth below, Me also represents methyl.)

Further, the chemical structure of2-[4-({5-[(2,6-difluoro-3,5-dimethoxybenzyl)oxy]pyrimidin-2-yl}amino)-1H-pyrazol-1-yl]ethanol (Compound B) is shown below.

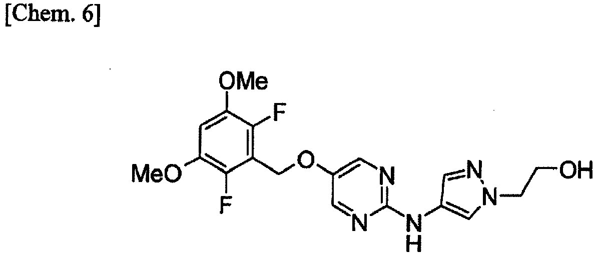

Further, the chemical structure of (2R)-3-[4-({5-[(2,6-difluoro-3,5-dimethoxybenzyl)oxy]pyrimidin-2-yl}amino)-1H-pyrazol-1-yl]propane-1,2-diol (Compound C) is shown below.

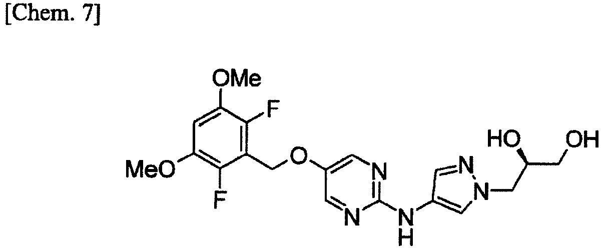

Further, the chemical structure of 5-[(2,6-difluoro-3,5-dimethoxybenzyl)oxy]-N-[4-(4-methylpiperazin-1-yl)phenyl]pyrimidin-2-amine (Compound D) is shown below.

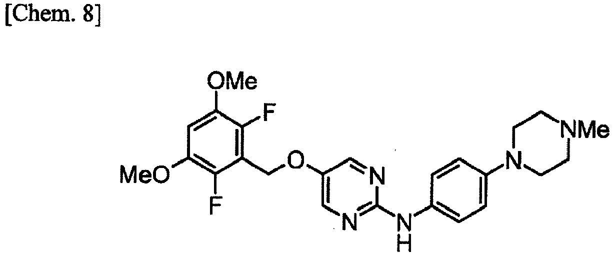

Further, the chemical structure of 5-[(2,6-difluoro-3,5-dimethoxybenzyl)oxy]-N-{1-methyl-5-[(4-methylpiperazin-1-yl)methyl]-1H-pyrazol-3-yl}pyrimidin-2-amine (Compound E) is shown below.

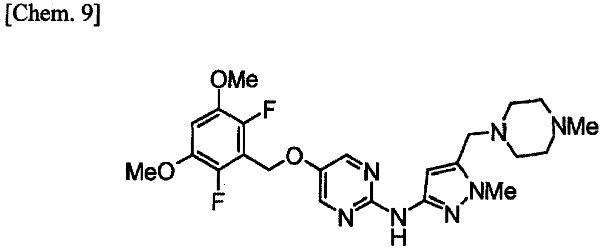

The "FGFR4-related cancer" refers to cancer in which activation of FGFR4 is involved in development or progression of cancer. For example, it refers to cancer with gene amplification of FGF19 which is a ligand of FGFR4 (Cancer Cell. 2011, 19(3): 347-58) or increase in expression of FGF19 (Oncogene. 2008 Jan 3; 27(1): 85-97); or cancer having activation mutations of FGFR4, for example, cancer having mutations from asparagines at position 535 to aspartic acid or lysine and from the valine at position 550 to leucine or glutaminic acid (J Clin Invest. 2009 Nov; 119(11): 3395-407), a mutation from tyrosine at position 376 to cysteine (Oncogene. 2010 Mar 11; 29(10): 1543-52.), or a mutation from proline at position 712 to threonine (Proc Natl Acad Sci U S A. 2013 Jul 8 Epub ahead of print PMID: 23836671) in FGFR4 isoform 1 defined in GenBank Accession Number: NM_002011. 3; or the like.

The "FGF19-related cancer" refers to cancer with gene amplification of FGF19 or increase in expression of FGF19. For the diagnosis of gene amplification, a plurality of methods including FISH (fluorescence in situ hybridization) method, methods using hybridization method such as an array comparison genomic hybridization method, Digital PCR (Polymerase Chain Reaction) method, PCR method, and the like can be used.

The "FGF 19 gene amplification-positive liver cancer" refers to liver cancer in the patients whose FGF19 gene region is amplified among the liver cancer patients. It is possible to diagnose the patients with FGF19 gene amplification-positive liver cancer, by using FGF19 mRNA and/or FGF19 protein as an index since the amount of the expressed FGF19 mRNA and/or FGF19 protein is increased as compared with patients having no FGF19 gene amplification or healthy individuals. As an example, the FGF19 mRNA expression can be measured by using a PCR method or nucleic acid hybridization method. The FGF19 protein in tissues can be detected by using immunohistochemical method or the like (PLoS One. 2012; 7 (5): e36713, Oncogene. 2008 Jan 3; 27 (1): 85-97), and FGF19 proteins in the blood can be detected by using Enzyme-Linked ImmunoSorbent Assay method or the like (BMC Cancer. 2012 Feb 6; 12 (1): 56).

Embodiments of the present invention will be shown below.
(1-1) A pharmaceutical composition for treating FGFR4-related cancer, comprising Compound A or a pharmaceutically acceptable salt thereof, and an excipient; in another embodiment, a pharmaceutical composition for treating FGF19-related cancer, comprising Compound A or a pharmaceutically acceptable salt thereof, and an excipient; and in still another embodiment, a pharmaceutical composition for treating FGF 19 gene amplification-positive liver cancer, comprising Compound A or a pharmaceutically acceptable salt thereof, and an excipient.
(1-2) Use of Compound A or a pharmaceutically acceptable salt thereof for the manufacture of a pharmaceutical composition for treating FGFR4-related cancer; in another embodiment, use of Compound A or a pharmaceutically acceptable salt thereof for the manufacture of a pharmaceutical composition for treating FGF19-related cancer; and in still another embodiment, use of Compound A or a pharmaceutically acceptable salt thereof for the manufacture of a pharmaceutical composition for treating FGF19 gene amplification-positive liver cancer.
(1-3) Use of Compound A or a pharmaceutically acceptable salt thereof for treating FGFR4-related cancer; in another embodiment, use of Compound A or a pharmaceutically acceptable salt thereof for treating FGF19-related cancer; and in still another embodiment, use of Compound A or a pharmaceutically acceptable salt thereof for treating FGF19 gene amplification-positive liver cancer.
(1-4) Compound A or a pharmaceutically acceptable salt thereof for treating FGFR4-related cancer; in another embodiment, Compound A or a pharmaceutically acceptable salt thereof for treating FGF19-related cancer; and in still another embodiment, Compound A or a pharmaceutically acceptable salt thereof for treating FGF19 gene amplification-positive liver cancer.
(1-5) A method for treating FGFR4-related cancer, comprising administering to a subject an effective amount of Compound A or a pharmaceutically acceptable salt thereof; in another embodiment, a method for treating FGF19-related cancer, comprising administering to a subject an effective amount of Compound A or a pharmaceutically acceptable salt thereof; and in still another embodiment, a method for treating FGF19 gene amplification-positive liver cancer, comprising administering to a subject an effective amount of Compound A or a pharmaceutically acceptable salt thereof.
(2-1) A pharmaceutical composition for treating FGFR4-related cancer, comprising Compound B or a pharmaceutically acceptable salt thereof, and an excipient; in another embodiment, a pharmaceutical composition for treating FGF19-related cancer, comprising Compound B or a pharmaceutically acceptable salt thereof, and an excipient; and in still another embodiment, a pharmaceutical composition for treating FGF19 gene amplification-positive liver cancer, comprising Compound B or a pharmaceutically acceptable salt thereof, and an excipient.
(2-2) Use of Compound B or a pharmaceutically acceptable salt thereof for the manufacture of a pharmaceutical composition for treating FGFR4-related cancer; in another embodiment, use of Compound B or a pharmaceutically acceptable salt thereof for the manufacture of a pharmaceutical composition for treating FGF19-related cancer; and in still another embodiment, use of Compound B or a pharmaceutically acceptable salt thereof for the manufacture of a pharmaceutical composition for treating FGF 19 gene amplification-positive liver cancer.
(2-3) Use of Compound B or a pharmaceutically acceptable salt thereof for treating FGFR4-related cancer; in another embodiment, use of Compound B or a pharmaceutically acceptable salt thereof for treating FGF19-related cancer; and in still another embodiment, use of Compound B or a pharmaceutically acceptable salt thereof for treating FGF19 gene amplification-positive liver cancer.
(2-4) Compound B or a pharmaceutically acceptable salt thereof for treating FGFR4-related cancer; in another embodiment, Compound B or a pharmaceutically acceptable salt thereof for treating FGF19-related cancer, and in still another embodiment, Compound B or a pharmaceutically acceptable salt thereof for treating FGF 19 gene amplification-positive liver cancer.
(2-5) A method for treating FGFR4-related cancer, comprising administering to a subject an effective amount of Compound B or a pharmaceutically acceptable salt thereof; in another embodiment, a method for treating FGF 19-related cancer, comprising administering to a subject an effective amount of Compound B or a pharmaceutically acceptable salt thereof; and in still another embodiment, a method for treating FGF19 gene amplification-positive liver cancer, comprising administering to a subject an effective amount of Compound B or a pharmaceutically acceptable salt thereof.
(3-1) A pharmaceutical composition for treating FGFR4-related cancer, comprising Compound C or a pharmaceutically acceptable salt thereof, and an excipient; in another embodiment, a pharmaceutical composition for treating FGF19-related cancer, comprising Compound C or a pharmaceutically acceptable salt thereof, and an excipient; and in still another embodiment, a pharmaceutical composition for treating FGF19 gene amplification-positive liver cancer, comprising Compound C or a pharmaceutically acceptable salt thereof, and an excipient.
(3-2) Use of Compound C or a pharmaceutically acceptable salt thereof for the manufacture of a pharmaceutical composition for treating FGFR4-related cancer; in another embodiment, use of Compound C or a pharmaceutically acceptable salt thereof for the manufacture of a pharmaceutical composition for treating FGF19-related cancer; and in still another embodiment, use of Compound C or a pharmaceutically acceptable salt thereof for the manufacture of a pharmaceutical composition for treating FGF19 gene amplification-positive liver cancer.
(3-3) Use of Compound C or a pharmaceutically acceptable salt thereof for treating FGFR4-related cancer; in another embodiment, use of Compound C or a pharmaceutically acceptable salt thereof for treating FGF19-related cancer; and in still another embodiment, use of Compound C or a pharmaceutically acceptable salt thereof for treating FGF19 gene amplification-positive liver cancer.
(3-4) Compound C or a pharmaceutically acceptable salt thereof for treating FGFR4-related cancer; in another embodiment, Compound C or a pharmaceutically acceptable salt thereof for treating FGF19-related cancer; and in still another embodiment, Compound C or a pharmaceutically acceptable salt thereof for treating FGF19 gene amplification-positive liver cancer.
(3-5) A method for treating FGFR4-related cancer, comprising administering to a subject an effective amount of Compound C or a pharmaceutically acceptable salt thereof; in another embodiment, a method for treating FGF19-related cancer, comprising administering to a subject an effective amount of Compound C or a pharmaceutically acceptable salt thereof; and in still another embodiment, a method for treating FGF19 gene amplification-positive liver cancer, comprising administering to a subject an effective amount of Compound C or a pharmaceutically acceptable salt thereof
(4-1) A pharmaceutical composition for treating FGFR4-related cancer, comprising Compound D or a pharmaceutically acceptable salt thereof, and an excipient; in another embodiment, a pharmaceutical composition for treating FGF19-related cancer, comprising Compound D or a pharmaceutically acceptable salt thereof, and an excipient; and in still another embodiment, a pharmaceutical composition for treating FGF 19 gene amplification-positive liver cancer, comprising Compound D or a pharmaceutically acceptable salt thereof, and an excipient.
(4-2) Use of Compound D or a pharmaceutically acceptable salt thereof for the manufacture of a pharmaceutical composition for treating FGFR4-related cancer; in another embodiment, use of Compound D or a pharmaceutically acceptable salt thereof for the manufacture of a pharmaceutical composition for treating FGF19-related cancer; and in still another embodiment, use of Compound D or a pharmaceutically acceptable salt thereof for the manufacture of a pharmaceutical composition for treating FGF 19 gene amplification-positive liver cancer.
(4-3) Use of Compound D or a pharmaceutically acceptable salt thereof for treating FGFR4-related cancer; in another embodiment, use of Compound D or a pharmaceutically acceptable salt thereof for treating FGF19-related cancer; and in still another embodiment, use of Compound D or a pharmaceutically acceptable salt thereof for treating FGF19 gene amplification-positive liver cancer.
(4-4) Compound D or a pharmaceutically acceptable salt thereof for treating FGFR4-related cancer; in another embodiment, Compound D or a pharmaceutically acceptable salt thereof for treating FGF19-related cancer; and in still another embodiment, Compound D or a pharmaceutically acceptable salt thereof for treating FGF 19 gene amplification-positive liver cancer.
(4-5) A method for treating FGFR4-related cancer, comprising administering to a subject an effective amount of Compound D or a pharmaceutically acceptable salt thereof; in another embodiment, a method for treating FGF19-related cancer, comprising administering to a subject an effective amount of Compound D or a pharmaceutically acceptable salt thereof; and in still another embodiment, a method for treating FGF19 gene amplification-positive liver cancer, comprising administering to a subject an effective amount of Compound D or a pharmaceutically acceptable salt thereof.
(5-1) A pharmaceutical composition for treating FGFR4-related cancer, comprising Compound E or a pharmaceutically acceptable salt thereof, and an excipient; in another embodiment, a pharmaceutical composition for treating FGF19-related cancer, comprising Compound E or a pharmaceutically acceptable salt thereof, and an excipient; and in still another embodiment, a pharmaceutical composition for treating FGF19 gene amplification-positive liver cancer, comprising Compound E or a pharmaceutically acceptable salt thereof, and an excipient.
(5-2) Use of Compound E or a pharmaceutically acceptable salt thereof for the manufacture of a pharmaceutical composition for treating FGFR4-related cancer; in another embodiment, use of Compound E or a pharmaceutically acceptable salt thereof for the manufacture of a pharmaceutical composition for treating FGF19-related cancer; and in still another embodiment, use of Compound E or a pharmaceutically acceptable salt thereof for the manufacture of a pharmaceutical composition for treating FGF 19 gene amplification-positive liver cancer.
(5-3) Use of Compound E or a pharmaceutically acceptable salt thereof for treating FGFR4-related cancer; in another embodiment, use of Compound E or a pharmaceutically acceptable salt thereof for treating FGF19-related cancer; and in still another embodiment, use of Compound E or a pharmaceutically acceptable salt thereof for treating FGF19 gene amplification-positive liver cancer.
(5-4) Compound E or a pharmaceutically acceptable salt thereof for treating FGFR4-related cancer; in another embodiment, Compound E or a pharmaceutically acceptable salt thereof for treating FGF19-related cancer; and in still another embodiment, Compound E or a pharmaceutically acceptable salt thereof for treating FGF 19 gene amplification-positive liver cancer.
(5-5) A method for treating FGFR4-related cancer, comprising administering to a subject an effective amount of Compound E or a pharmaceutically acceptable salt thereof; in another embodiment, a method for treating FGF19-related cancer, comprising administering to a subject an effective amount of Compound E or a pharmaceutically acceptable salt thereof; and in still another embodiment, a method for treating FGF19 gene amplification-positive liver cancer, comprising administering to a subject an effective amount of Compound E or a pharmaceutically acceptable salt thereof.

Furthermore, the pharmaceutically acceptable salt of Compound A, Compound B, Compound C, Compound D, or Compound E means an acid addition salt of Compound A, Compound B, Compound C, Compound D, or Compound E, and specific examples thereof include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditolyltartaric acid, citric acid, methanesulfonic acid, ethanesulfonic acid, benzenesulfonic acid, p-toluenesulfonic acid, aspartic acid, and glutamic acid. Further, "Compound A, Compound B, Compound C, Compound D, or Compound E" encompasses solvates, in particular, hydrates or ethanolates of Compound A, Compound B, Compound C, Compound D, or Compound E.

Further, in a certain embodiment of Compound A, Compound B, Compound C, Compound D, or Compound E, or a pharmaceutically acceptable salt thereof, the pharmaceutically acceptable salt includes a free base that does not form a salt, that is, for example, Compound A, Compound B, Compound C, Compound D, or Compound E, in another embodiment, it is Compound A, in still another embodiment, Compound B, in still another embodiment, Compound C, in still another embodiment, Compound D, and in still another embodiment, Compound E.

A pharmaceutical composition comprising Compound A, Compound B, Compound C, Compound D, or Compound E, or a pharmaceutically acceptable salt thereof as an active ingredient can be prepared using excipients that are usually used in the art, that is, excipients for pharmaceutical preparations, carriers for pharmaceutical preparations, and the like, according to the methods usually used.

Administration can be performed in the form of either oral administration via tablets, pills, capsules, granules, powders, solutions, and the like, or parenteral administration, such as injections such as intravenous injections, suppositories, transdermal solutions, transdermal patches, and transmucosal solutions.

The solid composition for oral administration is used in the form of tablets, powders, granules, or the like. In such a solid composition, one or more active ingredient(s) are mixed with at least one inactive excipient. In a conventional method, the composition may comprise inactive additives, such as a lubricant, a disintegrating agent, a stabilizer, or a solubilization assisting agent. If necessary, tablets or pills may be coated with a film of a sugar-coated substance or gastric- or enteric-soluble substance.

The liquid composition for oral administration comprises pharmaceutically acceptable emulsions, solutions, suspensions, syrups, elixirs, or the like, and also comprises generally used inert diluents, for example, purified water or ethanol. In addition to the inert diluent, the liquid composition may also comprise auxiliary agents such as a solubilization assisting agent, a moistening agent, and a suspending agent, sweeteners, flavors, aromatics, or antiseptics.

The injections for parenteral administration comprise sterile aqueous or nonaqueous solutions, suspensions, or emulsions. The aqueous solvent includes, for example, distilled water for injection or physiological saline. Examples of the nonaqueous solvent include alcohols such as ethanol. Such a composition may further comprise a tonicity agent, an antiseptic, a moistening agent, an emulsifying agent, a dispersing agent, a stabilizer, or a solubilization assisting agent. These are sterilized, for example, by filtration through a bacteria retaining filter, blending of a bactericide, or irradiation. In addition, these can also be used by preparing a sterile solid composition, and dissolving or suspending it in sterile water or a sterile solvent for injection prior to its use.

In the case of oral administration, the daily dose is generally from about 0.001 mg/kg to 100 mg/kg, preferably from 0.01 mg/kg to 30 mg/kg, and still more preferably from 0.1 mg/kg to 10 mg/kg, per body weight, administered in one portion or in 2 to 4 separate portions. In the case of intravenous administration, the daily dose is suitably administered from about 0.0001 mg/kg to 10 mg/kg per body weight, once a day or two or more times a day. In addition, a transmucosal agent is administered at a dose from about 0.001 mg/kg to 100 mg/kg per body weight, once a day or two or more times a day. The dose is appropriately decided in response to the individual case by taking the symptoms, the age, the gender, and the like into consideration.

Although varying depending on administration routes, formulations, administration sites, or the types of excipients or additives, the pharmaceutical composition of the present invention comprises 0.01% by weight to 100% by weight, and in a certain embodiment, 0.01% by weight to 50% by weight of one or more kinds of Compound A, Compound B, Compound C, Compound D, or Compound E, or a or a pharmaceutically acceptable salt thereof, which is an active ingredient.

The pharmaceutical composition of the present invention can be used in combination with various agents for treating or preventing cancer, particularly FGFR4-related cancer, FGF19-related cancer, FGF19 gene amplification-positive liver cancer, or liver cancer, for which the pharmaceutical composition is considered to be effective. The combined use may be administered simultaneously, or separately and continuously, or at a desired time interval. The preparations to be administered simultaneously may be a blend or may be prepared individually.

### Examples

Hereinbelow, the methods for preparing Compound A, Compound B, Compound C, Compound D, and Compound E are shown in Preparation Examples. Further, the methods for preparing these compounds are not limited to the preparation methods in the specific Preparation Examples shown below, and the compounds can be prepared by using a combination of the preparation methods or a method apparent to a person skilled in the art. In addition, these compounds can be prepared by the method described in Patent Document 4 as described above, or an analogous method thereto.

### Preparation Example 1 Preparation of Compound A

(1) A mixture of methyl 3,5-dimethoxybenzoate (1 g) and acetonitrile (20 mL) was ice-cooled, and N-fluoro-N'-(chloromethyl)triethylenediamine bis(tetrafluoroborate) (4.09 g) was added thereto, followed by stirring at room temperature overnight. To the reaction mixture, a saturated aqueous sodium hydrogen carbonate solution was added, followed by extraction with ethyl acetate. The organic layer was washed with saturated brine, and anhydrous magnesium sulfate and basic silica gel were added thereto, followed by stirring for 30 minutes and then filtering. The filtrate was concentrated under reduced pressure and the residue was then purified by silica gel column chromatography (ethyl acetate/hexane) to obtain methyl 2,6-difluoro-3,5-dimethoxybenzoate (292 mg).
   MS (ESI+): 233 [(M+H)⁺].
(2) A mixture of methyl 2,6-difluoro-3,5-dimethoxybenzoate (10 g) and tetrahydrofuran (57 mL) was ice-cooled, and lithium borohydride (3.0 mol/L tetrahydrofuran solution, 43 mL) was added thereto, followed by stirring at room temperature for 65 hours. The reaction mixture was ice-cooled again, and additional lithium borohydride (3.0 mol/L tetrahydrofuran solution, 14.4 mL) was added thereto, followed by stirring at room temperature for 22 hours. The reaction mixture was ice-cooled and slowly added into ice water (300 mL). Further, concentrated hydrochloric acid (25 mL) was slowly added thereto, followed by stirring at room temperature for 1 hour and extracting with toluene/ethyl acetate (1:1). The organic layer was washed with a saturated aqueous sodium bicarbonate solution and saturated brine, dried over anhydrous magnesium sulfate, and then filtered. The filtrate was concentrated under reduced pressure to obtain (2,6-difluoro-3,5-dimethoxyphenyl)methanol (8.67 g). MS (EI+): 204[(M)⁺].
(3) A mixture of (2,6-difluoro-3,5-dimethoxyphenyl)methanol (1.71 g), triethylamine (2.57 mL), and tetrahydrofuran (34.2 mL) was ice-cooled, and methanesulfonyl chloride (716 µL) was added thereto, followed by stirring for 1 hour. The insoluble materials were separated by filtration and then the filtrate was concentrated under reduced pressure. To the residue was added water, followed by extraction with ethyl acetate. The organic layer was washed with saturated brine, dried over anhydrous magnesium sulfate, and then filtered. The filtrate was concentrated under reduced pressure to obtain 2,6-difluoro-3,5-dimethoxybenzylmethanesulfonate (2.32 g).
   NMR (DMSO-d₆): 3.24 (3H, s), 3.88 (6H, s), 5.30 (2H, s), 7.12 (1H, t, J = 8.4 Hz).
(4) To a mixture of 2-chloro-5-hydroxypyrimidine (4.38 g), 2,6-difluoro-3,5-dimethoxybenzylmethanesulfonate (7.89 g), and N,N-dimethylformamide (78.9 mL) was added potassium carbonate (9.27 g), followed by stirring at 60°C for 1 hour. To the reaction mixture was added water, followed by stirring for 30 minutes under ice-cooling. The resulting solid was collected by filtration, washed with water, and then dried under reduced pressure to obtain 2-chloro-5-[(2,6-difluoro-3,5-dimethoxybenzyl)oxy]pyrimidine (8.53 g).
   MS (APCI/ESI+): 317 [(M+H)⁺].
(5) Under an argon atmosphere, to a mixture of 2-chloro-5-[(2,6-difluoro-3,5-dimethoxybenzyl)oxy]pyrimidine (1.03 g), 3-methoxy-4-[4-(4-methylpiperazin-1-yl)piperidin-1-yl]aniline (1.29 g), 1,1'-binaphthalene-2,2'-diyl bis(diphenylphosphine) (609 mg), cesium carbonate (3.19 g), and dioxane (20.6 mL) was added palladium acetate (146 mg) at room temperature, followed by stirring at 100°C for 4 hours. To the reaction mixture was added water, followed by extraction with ethyl acetate. The organic layer was washed with saturated brine, dried over anhydrous magnesium sulfate, and then filtered. The filtrate was concentrated under reduced pressure, and the residue was purified by silica gel column chromatography (chloroform/methanol/concentrated aqueous ammonia), purified by basic silica gel columnchromatography (ethyl acetate), and then solidified with ethyl acetate and then with ethanol to obtain 5-[(2,6-difluoro-3,5-dirnethoxybenzyl)oxy]-N-{3-methoxy-4-[4-(4-methylpiperazin-1-yl)piperidin-1-yl]phenyl}pyrimidin-2-amine (Compound A: 830 mg).
   MS (ESI+): 585 [(M+H)⁺].
   NMR (DMSO-d₆): 1.45-1.60 (2H, m), 1.73-1.84 (2H, m), 2.14 (3H, s), 2.17-2.58 (11H, m), 3.24-3.36 (2H, m), 3.75 (3H, s), 3.87 (6H, s), 5.16 (2H, s), 6.79 (1H, d, J = 8.8Hz), 7.07 (1H, t, J = 8.4Hz), 7.24 (1H, dd, J = 8.8, 2.4Hz), 7.32 (1H, d, J = 2.4Hz), 8.29 (2H, s), 9.21 (1H, s).

### Preparation Example 2 Preparation of Compound B

(1) Under an argon atmosphere, to a mixture of 2-chloro-5-[(2,6-difluoro-3,5-dimethoxybenzyl)oxy]pyrimidine (800 mg) prepared by the same method as in Preparation Example 1(4), 2-(4-amino-1H-pyrazol-1-yl)ethanol (642 mg), 1,1'-binaphthalene-2,2'-diyl bis(diphenylphosphine)(472 mg), cesium carbonate (2.47 g), and dioxane (16 mL) was added palladium acetate (113 mg) at room temperature, followed by stirring at 100°C for 6 hours. To the reaction mixture were added water and chloroform, the insoluble materials were separated by filtration, and the filtrate was then extracted with chloroform. The organic layer was washed with saturated brine, dried over anhydrous magnesium sulfate, and then filtered. The filtrate was concentrated under reduced pressure and the residue was purified by silica gel column chromatography (chloroform/methanol) to obtain 2-[4-({5-[(2,6-difluoro-3,5-dimethoxybenzyl)oxy]pyrimidin-2-yl}amino)-1H-pyrazol-1-yl]ethanol (Compound B: 139 mg).

MS (ESI+): 408 [(M+H)⁺].

NMR (DMSO-d₆): 3.69 (2H, dd, J = 11.0, 5.6Hz), 3.87 (6H, s), 4.07 (2H, t, J = 5.6Hz), 4.83 (1H, t, J = 5.4Hz), 5.14 (2H, s), 7.07 (1H, t, J = 8.4Hz), 7.45 (1H, d, J = 0.6Hz), 7.88 (1H, d, J = 0.6Hz), 8.26 (2H, s), 9.20 (1H, s).

### Preparation Example 3 Preparation of Compound C

(1) Under an argon atmosphere, to a mixture of 2-chloro-5-[(2,6-difluoro-3,5-dimethoxybenzyl)oxy]pyrimidine (1.33 g) prepared by the same method as in Preparation Example 1(4), 1-(tetrahydro-2H-pyran-2-yl)-1H-pyrazol4-amine(913 mg), 1,1'-binaphthalene-2,2'-diyl bis(diphenylphosphine) (785 mg), palladium acetate (189 mg), and dioxane (26.6 mL) was added cesium carbonate (4.11 g) at room temperature, followed by stirring at 100°C for 4 hours. To the reaction mixture was added water, followed by extraction with ethyl acetate. The organic layer was washed with saturated brine, dried over anhydrous sodium sulfate, and then filtered. The filtrate was concentrated under reduced pressure and the residue was purified by silica gel column chromatography (ethyl acetate/hexane) to obtain 5-[(2,6-difluoro-3,5-dimethoxybenzyl)oxy]-N-[1-(tetrahydro-2H-pyran-2-yl)-1H-pyrazol-4-yl]pyrimidin-2-amine (1.73 g).
   MS (APCI/ESI+): 448 [(M+H)⁺].
(2) To a mixture of 5-[(2,6-difluoro-3,5-dimethoxybenzyl)oxy]-N-[1-(tetrahydro-2H-pyran-2-yl)-1H-pyrazol-4-yl]pyrimidin-2-amine (3.59 g) and methanol (20 mL) was added hydrogen chloride (4 mol/L dioxane solution, 40 mL), followed by stirring at room temperature for 6 hours. The reaction mixture was concentrated under reduced pressure and then to the residue was added a saturated aqueous sodium bicarbonate solution. The resulting solid was collected by filtration, washed with diethyl ether, and dried under reduced pressure to obtain 5-[(2,6-difluoro-3,5-dimethoxybenzyl)oxyl-N-(1H-pyrazol-4-yl)pyrimidin-2-amine (2.9 g).
   MS (APCI/ESI+): 364 [(M+H)⁺].
(3) To a mixture of 5-[(2,6-difluoro-3,5-dimethoxybenzyl)oxy]-N-(1H-pyrazol-4-yl)pyrimidin-2-amine (50 mg), potassium carbonate (57 mg), and N,N-dimethylformamide (1 mL) was added [(4S)-2,2-dimethyl-1,3-dioxolan-4-yl]methyl 4-methylbenzenesulfonate (118 mg), followed by stirring at 60°C for 1 hour and at 110°C for 4 days. To the reaction mixture was added water, followed by extraction with ethyl acetate. The organic layer was washed with saturated brine, dried over anhydrous magnesium sulfate, and then filtered. The filtrate was concentrated under reduced pressure and the obtained residue was then purified by silica gel column chromatography (ethyl acetate/hexane) to obtain 5-[(2,6-difluoro-3,5-dimethoxybenzyl)oxy]-N-(1-{[(4R)-2,2-dimeihyl-1,3-dioxolan-4-yl]methyl}-1H-pyrazol-4-yl)pyrimidin-2-amine (39 mg).
   MS (APCI/ESI+): 478 [(M+H)⁺].
(4) To a mixture of 5-[(2,6-difluoro-3,5-dimethoxybenzyl)oxy]-N-(1-{[(4R)-2,2-dimethyl-1,3-dioxolan-4-yl]methyl}-1H-pyrazol-4-yl)pyrimidin-2-amine (45 mg) and tetrahydrofuran (2 mL) was added hydrochloric acid (1 mol/L, 1 mL), followed by stirring at 50°C for 3 hours. To the reaction mixture was added a saturated aqueous sodium bicarbonate solution, followed by extraction with chloroform. The organic layer was washed with saturated brine, dried over anhydrous sodium sulfate, and then filtered. The filtrate was concentrated under reduced pressure and the residue was purified by silica gel column chromatography (chloroform/methanol) and then solidified with ethyl acetate to obtain (2R)-3-[4-({5-[(2,6-difluoro-3,5-dimethoxybenzyl)oxy]pyrimidin-2-yl}amino)-1H-pyrazol-1-yl]propane-1,2-diol (Compound C: 25 mg).

MS (ESI+): 438 [(M+H)⁺].

NMR (DMSO-d₆):3.23-3.38 (2H, m), 3.72-3.80 (1H, m), 3.84-3.96 (7H, m), 4.15 (1H, dd, J = 13.8,4.1H), 4.67 (1H, t, J = 5.6Hz), 4.91 (1H, d, J = 5.3Hz), 5.14 (2H, s), 7.06 (1H, t, J = 8.4Hz), 7.45 (1H, d, J = 0.6Hz), 7.87 (1H, d, J = 0.6Hz), 8.26 (2H, s), 9.21 (1H, s).

### Preparation Example 4 Preparation of Compound D

(1) Under an argon atmosphere, to a mixture of 2-chloro-5-[(2,6-difluoro-3,5-dimethoxybenzyl)oxy]pyrimidine (150 mg) prepared by the same method as in Preparation Example 1(4), 4-(4-methylpiperazin-1-yl)aniline (109 mg), 1,1'-binaphthalene-2,2'-diyl bis(diphenylphosphine) (88 mg), cesium carbonate (463 mg), and dioxane (3 mL) was added palladium acetate (21 mg) at room temperature, followed by stirring at 100°C for 4 hours. To the reaction mixture was added water, followed by extraction with ethyl acetate. The organic layer was washed with saturated brine, dried over anhydrous magnesium sulfate, and then filtered. The filtrate was concentrated under reduced pressure and the residue was purified by silica gel column chromatography (chloroform/methanol/concentrated aqueous ammonia) and then solidified with ethyl acetate and diisopropyl ether to obtain 5-[(2,6-difluoro-3,5-dimethoxybenzyl)oxy]-N-[4-(4-methylpiperazin-1-yl)phenyl]pyrimidin-2-amine (Compound D: 156 mg).

MS (ESI+): 472 [(M+H)⁺].

NMR (DMSO-d₆): 2.21 (3H, s), 2.41-2.48 (4H, m), 2.98-3.08 (4H, m), 3.87 (6H, s), 5.15 (2H, s), 6.81-6.90 (2H, m), 7.07 (1H, t, J = 8.4Hz), 7.47-7.55 (2H, m), 8.26 (2H, s), 9.15 (1H, s).

### Preparation Example 5 Preparation of Compound E

(1) To a mixture of (1-methyl-3-nitro-1H-pyrazol-5-yl)methanol (398 mg), 3,4-dihydro-2H-pyrane (459 µL), and ethyl acetate (8 mL) was added p-toluenesulfonicacid monohydrate (96 mg), followed by stirring at room temperature for 1.5 hours. Further, 3,4-dihydro-2H-pyrane (459 µL) and p-toluenesulfonicacid monohydrate (96 mg) were added thereto, followed by stirring at room temperature for 1.5 hours. To the reaction mixture was added water, followed by extraction with ethyl acetate. The organic layer was washed with saturated brine, dried over anhydrous sodium sulfate, and then filtered. The filtrate was concentrated under reduced pressure and the residue was then purified by silica gel column chromatography (hexane/ethyl acetate) to obtain 1-methyl-3-nitro-5-[(tetrahydro-2H-pyran-2-yloxy)methyl]-1H-pyrazole (487 mg).
   MS (APC1/ESI+): 242 [(M+H)⁺].
(2) To a mixture of 1-methyl-3-nitro-5-[(tetrahydro-2H-pyran-2-yloxy)methyl]-1H-pyrazole (487 mg), tetrahydrofuran (4.9 mL), and ethanol (4.9 mL) was added 10% palladium-carbon (wet, 50 mg). Under a hydrogen atmosphere, the mixture was stirred for 12 hours and the insoluble materials were then separated by filtration. The filtrate was concentrated under reduced pressure to obtain 1-methyl-5-[(tetrahydro-2H-pyran-2-yloxy)methyl]-1H-pyrazol-3-amine (426 mg).
   MS (APCI/ESI+): 212 [(M+H)⁺].
(3) In the same manner as in Preparation Example 3(1), using 1-ethyl-5-[(tetrahydro-2H-pyran-2-yloxy)methyl]-1H-pyrazol-3-amine, and 2-chloro-5-[(2,6-difluoro-3,5-dimethoxybenzyl)oxy]pyrimidine prepared by the same method as in Preparation Example 1(4) as the starting materials, 5-[(2,6-difluoro-3,5-dimethoxybenzyl)oxy]-N-{1-methyl-5-[(tetrahydro-2H-pyran-2-yloxy)methyl]-1H-pyrazol-3-yl}pyrimidin-2-amine was obtained.
   MS (APCI/ESI+): 492 [(M+H)⁺].
(4) To a mixture of 5-[(2,6-difluoro-3,5-dimethoxybenzyl)oxy]-N-{1-methyl-5-[(tetrahydro-2H-pyran-2-yloxy)methyl]-1H-pyrazol-3-yl}pyrimidin-2-amine (706 mg) and methanol (8 mL) was added hydrogen chloride (4 mol/L dioxane solution, 8 mL), followed by stirring at room temperature for 3 hours. The reaction mixture was concentrated under reduced pressure and then to the residue was added saturated aqueous sodium bicarbonate solution, followed by stirring followed by extraction with chloroform. The organic layer was dried over anhydrous sodium sulfate, and then filtered. The filtrate was concentrated under reduced pressure, and the residue was purified by silica gel column chromatography (ethyl acetate/hexane) and then solidified with ethanol to obtain [3-({5-[(2,6-difluoro-3,5-dimethoxybenzyl)oxy]pyrimidin-2-yl}amino)-1-methyl-1H-pyrazol-5-yl]methanol (444 mg).
   MS (ESI+): 408 [(M+H)⁺].
(5) A mixture of [3-({5-[(2,6-difluoro-3,5-dimethoxybenzyl)oxy]pyrimidin-2-yl}amino)-1-methyl-1H-pyrazol-5-yl]methanol (350 mg), triethylamine (359 µL), dichloromethane (7 mL) and tetrahydrofuran (7 mL) was ice-cooled, and methanesulfonyl chloride (120 µL) was added thereto, followed by stirring at room temperature for 3 hours. To the reaction mixture were added water and ethyl acetate, and the resulting solid was collected by filtration and then dried under reduced pressure to obtain [3-({5-[(2,6-difluoro-3,5-dimethoxybenzyl)oxy]pyrimidin-2-yl}amino)-1-methyl-1H-pyrazol-5-yl]methylmethanesulfonate (218 mg).
   NMR (CDCl₃): 2.96 (3H, s), 3.85 (3H, s), 3.89 (6H, s), 5.16 (2H, s), 5.25 (2H, s), 6.68 (1H, t, J = 8.0Hz), 6.91 (1H, s), 7.63 (1H, brs), 8.24 (2H, s).
(6) To a mixture of [3-({5-[(2,6-difluoro-3,5-dimethoxybenzyl)oxy]pyrimidin-2-yl}amino)-1-methyl-1H-pyrazol-5-yl]methylmethanesulfonate (795 mg) and N-methylpyrrolidone (15.9 mL) was added 1-methylpiperazine (901 µL), followed by stirring at 80°C for 2 hours. To the reaction mixture were added water and a saturated aqueous sodium bicarbonate solution, the resulting solid was collected by filtration, and then the filtrate was extracted with chloroform. The organic layer was dried over anhydrous sodium sulfate and then filtered. The filtrate was concentrated under reduced pressure. Then, the residue and the solid previously collected by filtration were purified by silica gel columnchromatography (chloroform/methanol) and then by basic silica gel columnchromatography(ethyl acetate/methanol), and then solidified with ethanol/diisopropyl ether to obtain 5-[(2,6-difluoro-3,5-dimethoxybenzyl)oxy]-N-{1-methyl-5-[(4-methylpiperazin-1-yl)methyl]-1H-pyrazol-3-yl}pyrimidin-2-amine (Compound E: 168 mg).

MS (ESI+): 490 [(M+H)⁺].

NMR (DMSO-d₆): 2.14 (3H, s), 2.18-2.53 (8H, m), 3.45 (2H, s), 3.67 (3H, s), 3.87 (6H, s), 5.15 (2H, s), 6.46 (1H, s), 7.06 (1H, t, J = 8.4Hz), 8.26 (2H, s), 9.42 (1H, s).

The pharmacological activity of the pharmaceutical composition of the present invention was confirmed by Examples below.

### Test Example 1 FGFR4 Enzyme Assay

For an enzyme assay, human recombinant FGFR4 (CARNA BIOSCIENCES. Inc., Catalog No.; 08CBS-0716C) was used. The outline of the measurement method is shown below.

A test compound was dissolved in dimethyl sulfoxide (DMSO), and diluted with a reaction buffer (100 mM HEPES(pH 7.5), 0.003% Briji-35,0.004% Tween 20, 0.5 mM DTT, 10 mM MgCl₂) to give a DMSO concentration of 2% to prepare a compound solution. Into a well of a 384-well plate,
(1) 4 µL of a compound solution was added,
(2) 2 µL of an FGFR4 enzyme (3ng/µL) diluted with a reaction buffer was added,
(3) after 20 minutes, 4 µL of an aqueous solution of fluorescent substrate peptide (composition: 100 mM HEPES (pH 7.5), 0.003% Brij-35, 0.004% Tween 20, 0.5 mM DTT, 10 mM MgCl2, 3.75 µM substrate-FL-peptide 30 (Perkin Elmer Product NO. 760430), 750 µM ATP) was added, and
(4) the mixture was allowed to undergo a reaction at room temperature for 30 minutes (concentration of the test compound species during assay of 10 nM).
(5) 15 µL/well of a 20 mM EDTA solution was added thereto to stop the reaction, and then in a LabChip EZ Reader (Perkin Elmer), the amount of a phosphorylated substrate peptide produced by the kinase reaction of FGFR4 was measured.

By taking the measurement value of the well to which the test compound had not been added (only DMSO had been added) as 0% inhibition and taking the measurement value of the well to which the FGFR4 enzyme had not been added as 100% inhibition, the FGFR4 enzyme inhibitory rate of each compound at 10 nM was calculated. That is, the FGFR4 enzyme inhibitory rate was calculated by <FGFR4 enzyme inhibitory rate (%)> = [1 - {<the measured value of the well to which the test compound has been added> - <the measured value of the well to which the FGFR4 enzyme has not been added>} / {<the measured value of the well to which the test compound has not been added (only DMSO has been added)> - < the measured value of the well to which the FGFR4 enzyme has not been added>}] x 100. The results are shown in Table 1.

**[Table 1]**

| FGFR4 Kinase Activity Inhibitory Rate of Each Test Compound (10 nM) | |
|---|---|
| Test compound | Inhibitory rate (%) |
| Compound A | 78 |
| Compound B | 65 |
| Compound C | 71 |
| Compound D | 82 |
| Compound E | 31 |

From the results above, it was confirmed that Compound A, Compound B, Compound C, Compound D, and Compound E, which are the active ingredients of the pharmaceutical compositions of the present invention, inhibit the kinase activity of FGFR4.

### Test Example 2 Growth Assay of FGF19 Gene Amplification-Positive Liver Cancer Cell (HuH-7)

The HuH-7 cell is a hepatocellular carcinoma cell line which is reported to have FGF19 gene amplification (Cancer Cell. 2011, 19(3): 347-58).

The HuH-7 cell (purchased from Human Science Research Resources Bank, JCRB 0403) was seeded into a 96-well polystyrene flat-bottom plate at 500 cells/90 µL/well, and on the next day, a test compound solution (10 µL) was added thereto (final concentration of the test compound solution: 37 nM, and final concentration of DMSO: 0.1 %). After 5 days from the addition of the test compound solution, the number of HuH-7 cells were measured with ARVOx3 (Perkin Elmer) using a CellTiter-Glo Luminescent Cell Viability Assay (Promega). By taking the measured value of the well to which the test compound had not been added (only DMSO had been added) as 0% inhibition and taking the measured value of the well without seeding (only medium present) as 100% inhibition, the growth inhibitory rate of each test compound at 37 nM was calculated. That is, the inhibitory rate was calculated by <the growth inhibitory rate (%)> = <1 - (the measured value of the well to which the test compound was added> - <the measured value of the well to which the cells were not added (only medium present)>) / {<the measured value of the well to which the test compound was not added (only DMSO was added)> - <the measured value of the well to which the cells were not added (only medium present)}] × 100. The results are shown in Table 2.

**[Table 2]**

| HuH-7 Cell Growth Inhibitory Rate of Each Test Compound (37 nM) | |
|---|---|
| Test compound | Inhibitory rate (%) |
| Compound A | 53 |
| Compound B | 47 |
| Compound C | 55 |
| Compound D | 65 |
| Compound E | 11 |

From the results above, it was confirmed that Compound A, Compound B, Compound C, Compound D, and Compound E, which are the active ingredients of the pharmaceutical compositions of the present invention, inhibit the growth of the liver cancer cells having FGF19 gene amplification.

### Test Example 3 Antitumor Test using Mouce Model Subcutaneously Transplanted with FGF 19 Gene Amplification-Positive Liver Cancer Cell (HuH-7)

HuH-7 cells at 3×10⁶ cellos/0.1 mL (solution suspended in PBS and Matrigel at 1:1) was subcutaneously transplanted to nude mice (CanN.Cg-Foxnlnu/CrlCrlj(nu/nu): Charles River Laboratories Japan, Inc., male, 4-5 week old). After about 3 weeks after the transplantation, the mice were grouped according to the tumor volumes and body weights. Each test compound-administered group was composed with 4 or 5 mice, and the average tumor volume of each group was 147 mm³ to 180 mm³. The test compound was made into a suspension using a 0.5% aqueous methyl cellulose solution and orally administered once a day daily. The administration dose of the test compound was 0.3 mg/kg/day for Compound C, and 1 mg/kg/day for Compound A, Compound B, Compound D, and Compound E. On the administration initiation day (0^{th} day) and the final day (the 9^{th} day for Compound E, the 10^{th} day for Compound A, Compound C, and Compound D, and the 13^{th} day for Compound B), the tumor volume was measured. For the tumor volume, a short diameter and long diameter were measured using a calipers, and the tumor volume was calculated by (tumor volume mm³) = (short diameter mm x short diameter mm x long diameter mm)/2. Further, the group to which the test compound was not administered (that is, only a 0.5% aqueous methyl cellulose solution had been administered) was taken as a control group, and the final day of the control group was set to be the same days as that of each compound (the 9^{th} day for Compound E, the 10^{th} day for Compound A, Compound C, and Compound D, and the 13^{th} day for Compound B). The tumor growth inhibitory rate of test compound was calculated by <the tumor growth inhibitory rate (%)> = <1 - (increase in the tumor volume of the test compound-administered group / increase in the tumor volumes of the control group)> x 100. The increase in the tumor volume was calculated by (increase in the tumor volume) = (the tumor volume on the final day of each group) - (the tumor volume on the 0^{th} day of each group). The results are shown in Table 3.

**[Table 3]**

| Tumor Growth Inhibitory Rate in Mice Models Subcutaneously Transplanted with HuH-7 Cells | |
|---|---|
| Test compound | Tumor growth inhibitory rate (%) |
| Compound A | 80 |
| Compound B | 80 |
| Compound C | 56 |
| Compound D | 67 |
| Compound E | 44 |

From the results above, it was confirmed that Compound A, Compound B, Compound C, Compound D, and Compound E, which are the active ingredients of the pharmaceutical compositions of the present invention, inhibit the growth of the liver cancer in animal models subcutaneously transplanted with FGF19 gene amplification-positive liver cancer cells.

From the results of Test Examples 1 to 3, it was demonstrated that Compound A, Compound B, Compound C, Compound D, or Compound E or a pharmaceutically acceptable salt thereof inhibits the FGFR4 kinase activity and is useful in growth inhibition in various types of cancer related to FGFR4 or FGF19, in particular liver cancer, and in a certain embodiment, FGF19 gene amplification-positive liver cancer.

### Industrial Applicability

Compound A, Compound B, Compound C, Compound D, or Compound E, or a pharmaceutically acceptable salt thereof, which is the active ingredient of the pharmaceutical composition of the present invention, has an inhibitory action on FGFR4, and can be used as an active ingredient of a pharmaceutical composition for treating FGFR4-related cancer, in another embodiment, a pharmaceutical composition for treating FGF19-related cancer, and in still another embodiment, a pharmaceutical composition for treating FGF19 gene amplification-positive liver cancer.

## Claims

1. A pharmaceutical composition for treating FGFR4-related cancer, comprising 5-[(2,6-difluoro-3,5-dimethoxybenzyl)oxy]-N-{3-methoxy-4-[4-(4-methylpiperazin-1-yl)piperidin-1-yl]phenyl}pyrimidin-2-amine, 2-[4-({5-[(2,6-difluoro-3,5-dimethoxybenzyl)oxy]pyrimidin-2-yl}amino)-1H-pyrazol-1-yl]ethanol, (2R)-3-[4-({5-[(2,6-difluoro-3,5-dimethoxybenzyl)oxy]pyrimidin-2-yl}amino)-1H-pyrazol-1-yl]propane-1,2-diol, 5-[(2,6-difluoro-3,5-dimethoxybenzyl)oxy]-N-[4-(4-methylpiperazin-1-yl)phenyl]pyrimidin-2-amine or 5-[(2,6-difluoro-3,5-dimethoxybenzyl)oxy]-N-{1-methyl-5-[(4-methylpiperazin-1-yl)methyl]-1H-pyrazol-3-yl}pyrimidin-2-amine, or a pharmaceutically acceptable salt thereof, and an excipient.

2. The pharmaceutical composition according to claim 1, wherein the FGFR4-related cancer is FGF19-related cancer.

3. The pharmaceutical composition according to claim 1, wherein the FGFR4-related cancer is FGF19 gene amplification-positive liver cancer.

4. The pharmaceutical composition according to any one of claims 1 to 3, comprising 5-[(2,6-difluoro-3,5-dimethoxybenzyl)oxy]-N-{3-methoxy-4-[4-(4-methylpiperazin-1-yl)piperidin-1-yl]phenyl}pyrimidin-2-amine or a pharmaceutically acceptable salt thereof, and an excipient.

5. The pharmaceutical composition according to any one of claims 1 to 3, comprising 2-[4-({5-[(2,6-difluoro-3,5-dimethoxybenzyl)oxy]pyrimidin-2-yl}amino)-1H-pyrazol-1-yl]ethanol or a pharmaceutically acceptable salt thereof, and an excipient.

6. The pharmaceutical composition according to any one of claims 1 to 3, comprising (2R)-3-[4-({5-[(2,6-difluoro-3,5-dimethoxybenzyl)oxy]pyrimidin-2-yl}amino)-1H-pyrazol-1-yl]propane-1,2-diol or a pharmaceutically acceptable salt thereof, and an excipient.

7. The pharmaceutical composition according to any one of claims 1 to 3, comprising 5-[(2,6-difluoro-3,5-dimethoxybenzyl)oxy]-N-[4-(4-methylpiperazin-1-yl)phenyl]pyrimidin-2-amine or a pharmaceutically acceptable salt thereof, and an excipient.

8. The pharmaceutical composition according to any one of claims 1 to 3, comprising 5-[(2,6-difluoro-3,5-dimethoxybenzyl)oxy]-N-{1-methyl-5-[(4-methylpiperazin-1-yl)methyl]-1H-pyrazol-3-yl}pyrimidin-2-amine or a pharmaceutically acceptable salt thereof, and an excipient.
